# EUROPÄISCHE PATENTANMELDUNG

(11) **EP 0 572 934 A1**
(43) Veröffentlichungstag der Anmeldung: **08.12.1993**
(21) Anmeldenummer: 93108640.9
(22) Anmeldetag: 28.05.1993
(51) Int. Cl.: A61M 25/04, A61M 5/46

(54) **Leitung zum Einführen in ein Hohlorgan**

(30) Priorität: 02.06.1992 DE 4218144
(71) Anmelder: Kaltenbach, Martin, Prof. Dr. med., D-63303 Dreieich (DE)
(72) Erfinder: Kaltenbach, Martin, Prof. Dr. med., D-63303 Dreieich (DE)
(74) Vertreter: Patent- und Rechtsanwaltssozietät, Schmitt, Maucher & Börjes

(57) **Zusammenfassung**

Eine Leitung (1), die ein Schlauch und insbesondere ein Katheter sein kann, kann dadurch in einem Hohlorgan und insbesondere in einem Blutgefäß (3) mit einem vorbestimmten Abstand ihrer Mündung (4) von ihrem Eintritt (5) in das Hohlorgan verankert werden, daß sie etwa in dem Abstand ihrer Verankerungstiefe eine seitliche Lochung (6) und in ihrer Innenlängshöhlung (2) einen Verankerungs-Faden (7) aus federelastischem Werkstoff hat. Der Faden (7) ist im Bereich der Mündung (4), also in Einführrichtung hinter der Lochung (6) bzw. in dem Bereich zwischen dieser Lochung (6) und der Mündung (4) U-förmig gekrümmt und tritt mit einem Halteende (8) durch die seitliche Lochung (6) aus. Der zwischen der Lochung (6) und der Mündung (4) liegende Bereich der Leitung (1) wird in seiner U- oder V-förmigen Lage zusammengehalten, wobei die eigentliche Krümmung unter Umständen sogar vor oder außerhalb der Mündung (4) liegen könnte. Das dem Halteende (8) entgegengesetzte andere Ende (9) des Fadens (7) ragt aus dem außerhalb des Hohlorganes befindlichen Bereich der Leitung (1) hervor, so daß zum Lösen der Verankerung an diesem Ende (9) so lange gezogen werden kann, bis die U- oder V-förmige Schlinge aufgelöst und das Halteende (8) zurückgezogen sind. Gegebenenfalls kann dabei der Faden auch doppelt liegen und an seinem Halteende (8) umgebogen sein, so daß sich im Mündungsbereich zwei schlingenförmige Lagen ergeben.

## Beschreibung

Die Erfindung betrifft eine Leitung mit einer Innenlängshöhlung, insbesondere Schlauch, zum Einführen in ein Hohlorgan, zum Beispiel in ein Blutgefäß, in die Harnblase oder dergleichen, wobei zwischen der Mündung der Leitung und ihrem Eintritt in das Hohlorgan in Gebrauchsstellung ein vorbestimmter Abstand vorgesehen ist.

Es ist bereits bekannt, den Schlauch eines Blasenkatheters mit seiner Mündung im Inneren der Blase mit Hilfe eines Federelementes zu verankern, welches während der Einführung durch den Harnleiter durch dessen Wandungen zusammengedrückt wird und sich nach Verlassen des Harnleiters in der Blase aufgrund der Federwirkung aufspreizt. Zum Zurückziehen ist ein entsprechendes Werkzeug, zum Beispiel ein spezieller Mandrin erforderlich, mit welchem die Federwirkung und die Aufspreizung rückgängig gemacht werden können.

Häufig ist es jedoch erforderlich, eine Leitung oder einen Schlauch durch eine künstliche Öffnung in ein Hohlorgan einzuführen und die Leitung zu verankern. Vor allem bei Blutgefäßen ist dies ein häufiger Vorgang. Dabei sind Einführbestecke bekannt, die nach dem Punktieren mit einer Kanüle über diese geschoben werden und dann auch die Öffnung in dem Blutgefäß abdichten. Durch derartige zu dem Einführbesteck gehörende Einführhülsen können Katheter oder ein festzulegender Schlauch in das Blutgefäß eingeführt werden.

Dabei ist es wünschenswert, die Einführtiefe in definierter und bekannter Weise festzulegen und den eingeführten Schlauch zugfest aber lösbar zu verankern. Beispielsweise soll ein Schlauch in ein Hohlorgan mit genau definierter Tiefe eingeführt werden können, wenn er als Infusionsschlauch benötigt wird und ein zu tiefes Einschieben die Gefahr einer Perforation zum Beispiel im Herzbereich bedeuten könnte. Umgekehrt darf jedoch auch die Einführtiefe nicht zu gering sein.

Es besteht deshalb die Aufgabe, eine Leitung der eingangs erwähnten Art zu schaffen, die in ein Hohlorgan einführbar und dort lösbar verankerbar ist, aber auch leicht wieder zurückgezogen werden kann, ohne daß ein besonderes Werkzeug oder ein Mandrin erforderlich sind. Dabei soll eine definierte Einführtiefe fixiert werden können.

Die Lösung dieser Aufgabe besteht darin, daß die Leitung etwa in dem vorbestimmten Abstand von ihrer Mündung eine Öffnung hat, und daß zum axialen Fixieren der Leitung im Eintritt ein elastisches Element vorgesehen ist, das durch die Öffnung hindurch sich im Hohlorgan abstützt und das durch eine axiale Relativbewegung zwischen Leitung und elastischem Element die Leitung gegenüber dem Hohlorgan wieder freigibt.

Besonders zweckmäßig ist es dabei, wenn die Leitung etwa in dem Abstand von ihrer Mündung, in dem sie in Gebrauchsstellung in das Hohlorgan ragt, wenigstens eine seitliche Lochung oder Öffnung hat und daß zum Fixieren der Leitung ein im Bereich der Mündung der Leitung U-förmig gekrümmter Faden aus federelastischem Werkstoff vorgesehen ist, der mit einem Halteende durch die seitliche Lochung austritt, von dem zwischen dieser Lochung und der Mündung liegenden Bereich der Leitung U- oder V-förmig liegend zusammengehalten ist und mit dem anderen Ende an dem außerhalb des Hohlorganes befindlichen Bereich der Leitung übersteht.

Durch diese Merkmale und Maßnahmen ist es möglich, die Leitung oder den Schlauch mit dem darin befindlichen, im Mündungsbereich U-förmig gekrümmt liegenden Faden durch eine Einführhülse in das Hohlorgan oder Blutgefäß einzuschieben, bis der aus der seitlichen Öffnung überstehende Fadenteil und dessen Halteende vollständig in das Hohlorgan oder Blutgefäß eingetreten ist. Nach Entfernen der Einführhülse kann die Leitung oder der Schlauch zusammen mit dem Faden zurückgezogen werden, wobei der abstehende Faden mit der Leitung oder dem Schlauch einen Widerhaken bildet und ein Zurückziehen verhindert, wenn die seitliche Öffnung und der daraus vorstehende Faden an die Innenseite des Hohlorganes oder Blutgefäßes anschlägt. Da der Faden aus federelastischem Werkstoff besteht, kann er die entsprechende Verankerung bewirken.

Soll die Leitung oder der Schlauch wieder aus dem Hohlorgan entfernt werden, genügt es, an dem außen überstehenden Ende des Fadens zu ziehen, wodurch der U- oder V-förmige Bereich dieses Fadens innerhalb der Leitung zurückgezogen und aufgefaltet wird, so daß dann auch das Halteende durch die Öffnung in das Innere der Leitung oder des Schlauches und schließlich daraus herausgezogen werden kann. Der Faden kann auch zur Lösung der Verankerung vor dem Herausziehen zuerst nach vorne bzw. nach innen geschoben werden.

Diese Verankerungsanordnung erlaubt es also, eine Leitung oder einen Schlauch in einem Hohlorgan mit vorbestimmter Tiefe zu verankern, so daß einerseits bei Verwendung des Schlauches als Infusionsschlauch ein zu tiefes Einschieben und damit die Gefahr von Perforationen im Herzbereich vermieden werden oder der Schlauch als Meßvorrichtung benutzt werden kann, um zum Beispiel den Abstand des Hohlorganges oder Blutgefäßes von der Hautoberfläche festzustellen, ohne daß es dabei zu nachteiligen Meßfehlern kommen kann.

Vor allem die Bestimmung des Abstandes des Hohlorganes unter der Hautoberfläche ist dann wichtig, wenn eine Arterie nach einer Punktion wieder geschlossen werden muß und dazu ein Kollagenverschluß auf die Öffnung der Arterie gebracht werden soll. Dabei muß nämlich das Kollagen in genau definierter Tiefe unter der Haut oberhalb der Arterie abgesetzt werden, damit es nicht etwa in die Arterie hineingelangt und die Gefahr eines Gefäßverschlusses bewirkt oder aber aufgrund einer zu großen Entfernung von der Arterie keine Verschlußwirkung ausübt. Mit dem erfindungsgemäß verankerten Schlauch kann nun der Operateur beispielsweise anhand einer Oberflächenmarkierung an dem Schlauch erkennen, in welcher Tiefe sich die punktierte Arterie befindet. Entsprechend tief kann er dann eine Vorrichtung zum Abgeben des Kollagenverschlusses einführen, nachdem der "Meßschlauch" entfernt ist.

Eine Verbesserung der Verankerung kann darin bestehen, daß der Faden über seine Länge und außerhalb der Leitung und der seitlichen Lochung doppelt verläuft und an seinem Halteende insbesondere U- oder V-förmig umgebogen oder geknickt ist. Somit ergeben sich im Mündungsbereich statt zweier durch die U-förmige Umbiegung gebildeter Fadenstränge in diesem Bereich deren vier. Das Halteende ist aber im Gegensatz zu nur einem einlagigen Faden aufgrund der Doppellagigkeit und der dort vorgesehenen Umbiegung oder Knickung glatt und kann sich nicht in die Gefäßinnenwand bohren.

Um die Leitung mit unterschiedlichen Einführtiefen benutzen zu können, können mehrere seitliche Lochungen in unterschiedlichen Abständen zur Mündung vorgesehen sein. Somit kann der Operateur zuvor wählen, wie tief die Leitung in ein Hohlorgan hineinragen soll, indem er den Verankerungs-Faden und dessen Verankerungsende an einer entsprechenden seitlichen Lochung austreten läßt.

Zur Verbesserung der Verankerung insbesondere auch mit mehreren Fäden kann es vorteilhaft sein, wenn mehrere seitliche Lochungen etwa in gleichem Abstand am Umfang verteilt und/oder in unterschiedlichen Abständen zur Mündung vorgesehen sind.

Die erfindungsgemäß gestaltete Leitung kann mit dem entsprechend vorbereiteten Faden in bekannter Weise durch ein Führungsbesteck bzw. eine Führungshülse in eine Arterie eingeführt werden, wonach das Führungsbesteck entfernt wird, während die Leitung oder der Schlauch, vorzugsweise ein Katheter, aufgrund des Halteendes des Verankerungsfadens in der Arterie hängenbleibt.

Dabei kann die Leitung an ihrer Außenseite markierbar sein oder eine Längenmarkierung tragen. Dies hat den Vorteil, daß zum Verschließen der Punktierungsöffnung an dem Blutgefäß eine Einführhülse für Kollagen so weit vorgeschoben werden kann, bis sie kurz vor dem Eingang in die Arterie endet. Nunmehr kann der elastische, aber eine Eigensteifigkeit aufweisende Faden, zum Beispiel ein Nylon-Faden zurückgezogen werden, bis sein Halteende in geradliniger Fortsetzung zu dem entgegengesetzten Ende verläuft, wonach der Faden in die Arterie vorgeschoben werden kann. Danach kann die Leitung, der Schlauch oder Katheter entfernt werden und nun der Verschlußstöpsel aus Kollagenmaterial durch die Einführhülse über den Faden bis vor die Arterienöffnung geführt werden. Anstelle des Fadens kann jedoch auch ein in die Einführhülse eingeführter Draht verwendet werden.

Es sei noch erwähnt, daß die Leitung mehrere Verankerungsfäden enthalten kann, von denen jeweils ein Halteende durch dieselbe oder unterschiedliche seitliche Lochungen austreten kann. Entsprechend kann die Verankerung verbessert werden.

Eine weitere Ausgestaltung der Leitung von ganz erheblicher Bedeutung kann von vorneherein eine Abdichtung ihres Eintrittsbereiches gegenüber dem Durchtritt in das Hohlorgan vorsehen und dadurch gekennzeichnet sein, daß die Leitung in ihrem Inneren einen lösbar oder entnehmbar angeordneten Ballonkatheter enthält, dessen Ballonbereich in Gebrauchsstellung wenigstens teilweise aus der in dem Hohlorgan befindlichen Mündung austritt. Wird der Ballon in dieser Position aufgeweitet, dichtet er die Punktionsstelle in dem Blutgefäß von innen her ab und unterbindet somit den Blutzufluß, bis zum Beispiel der schon erwähnte Kollagenverschluß gesetzt ist, ohne daß während der Anbringung des Kollagenverschlusses die Arterie durch Abdrücken verschlossen werden muß. Ein solches Abdrücken der Arterie ist nämlich unangenehm und kann das Gewebe im Operationsbereich so verschieben, daß eine vorher abgemessene Tiefe unter Umständen nicht mehr zutreffend ist. Ferner sind seitliche Verschiebungen möglich, so daß der Verschluß die gewünschte Stelle nicht mit Sicherheit erreicht. Wird jedoch die Punktionsöffnung durch den erwähnten Ballonkatheter verschlossen, kann das Kollagen problemlos und ohne die geschilderten Nachteile an der günstigsten Stelle plaziert werden. Dabei kann der Kollagenverschluß in bekannter Weise zunächst röhrchenförmig gestaltet sein und, wie schon bei dem vorhergehenden Ausführungsbeispiel beschrieben, über die Katheterzufuhr eingefädelt und zugeführt werden.

Insgesamt ergibt sich eine Verankerung einer Leitung, eines Schlauches oder eines Katheters mit Hilfe eines schlingenförmig im Inneren verlaufenden und an einer seitlichen Öffnung austretenden Fadens, die preiswert und dennoch sehr effektiv ist und nicht nur eine feste Verankerung eines Katheters, sondern auch eine genau definierte Tiefe der Eintrittslänge dieses Katheters in ein Hohlorgan ergibt und damit ermöglicht, die Leitung, den Schlauch oder den Katheter zum Abmessen des Abstandes der Eintrittsöffnung von der Hautoberfläche zu verwenden, um so einen Kollagenverschluß präzise setzen zu können.

Nachstehend sind Ausführungsbeispiele der Erfindung anhand der Zeichnung näher beschrieben. Es zeigt in zum Teil schematisierter Darstellung:
- Fig.1: einen Längsschnitt durch einen punktierten Teil eines Blutgefäßes mit einem darin eingeführten schlauchförmigen Leitungsstück, in dessen Innerem ein Faden zur Verankerung angeordnet ist, dessen Halteende aus einer seitlichen Öffnung der Leitung vorsteht, nachdem die Leitung durch eine Punktionsöffnung des Gefäßes über das Halteende des Fadens hinaus eingeführt ist,
- Fig.2: eine etwa Fig.1 entsprechende Darstellung, wobei die Leitung nunmehr so weit zurückgezogen ist, daß das Halteende im Bereich der seitlichen Öffnung oder Lochung der Leitung von innen her an der Wandung des Blutgefäßes widerhakenartig anschlägt und ein weiteres Zurückziehen der Leitung verhindert,
- Fig.3: eine den Fig.1 und 2 entsprechende Darstellung, wobei der Verankerungsfaden durch Zug an seinem außenliegenden Ende so weit zurückgezogen ist, daß die im Mündungsbereich befindliche Umbiegung aufgelöst und das Halteende bereits teilweise nach innen zurückgezogen ist,
- Fig.3a: eine Fig.3 entsprechende Darstellung, wobei das Halteende des Verankerungsfadens durch ein Einschieben des Fadens aus der seitlichen Öffnung in das Innere der Leitung und sogar aus deren Mündung herausbewegt wird, bevor der Verankerungsfaden dann zurückgezogen werden kann,
- Fig.4: eine Abwandlung der Führung des Verankerungsfadens, der doppeltgelegt ist und an dessen Halteende die beiden Stränge durch eine Umbiegung verbunden sind,
- Fig.5: eine Einführhülse nach ihrem Eintritt in ein Blutgefäß, mit deren Hilfe die Leitung in die in Fig.1 und 2 dargestellte Lage gebracht werden soll,
- Fig.6: das Einführen der Leitung mit ihrem Verankerungsfaden durch die Einführhülse gem. Fig. 5,
- Fig.7: die Anordnung der Leitung nach Entfernen der Einführhülse, wobei in diesem Ausführungsbeispiel im Inneren der Leitung noch eine Elektrode oder ein Werkzeug zum Erweitern einer Gefäßverengung oder dergleichen angeordnet ist,
- Fig.8: eine auf die Außenseite der mit einer Tiefenmarkierung versehenen Leitung aufgerschobene Einführhülse und
- Fig.9: die Einführhülse mit einem in ihrem Inneren verlaufenden Führungsdraht, über welchen ein hülsenförmiger Kollagenverschluß zuführbar ist,
- Fig.10: eine Einführhülse, durch welche eine Leitung mit in deren Innerem angeordneten Ballonkatheter in ein Blutgefäß eingeführt wird,
- Fig.11: die mit Hilfe eines Fadens verankerte Leitung gem. Fig.10 in Gebrauchsstellung, wobei für den Verschluß der Punktionsstelle und zum Unterbinden der Blutzufuhr der Ballon stromaufwärts entfaltet ist,
- Fig.12: eine Abwandlung der Fig.13, bei welcher der Ballon unmittelbar an der Mündung der Leitung und im Punktionsbereich aufgefaltet ist,
- Fig.13: eine der Fig.9 entsprechende Darstellung, bei welcher die Leitung entfernt ist und ein Kollagenstöpsel mit Hilfe einer Einführhülse zuführbar ist, wobei das Blutgefäß noch von dem Ballonkatheter verschlossen ist, sowie
- Fig.14: die Gebrauchslage des Kollagenverschlusses und die Zurückziehung des Ballonkatheters, nachdem der Ballon wieder entleert ist.

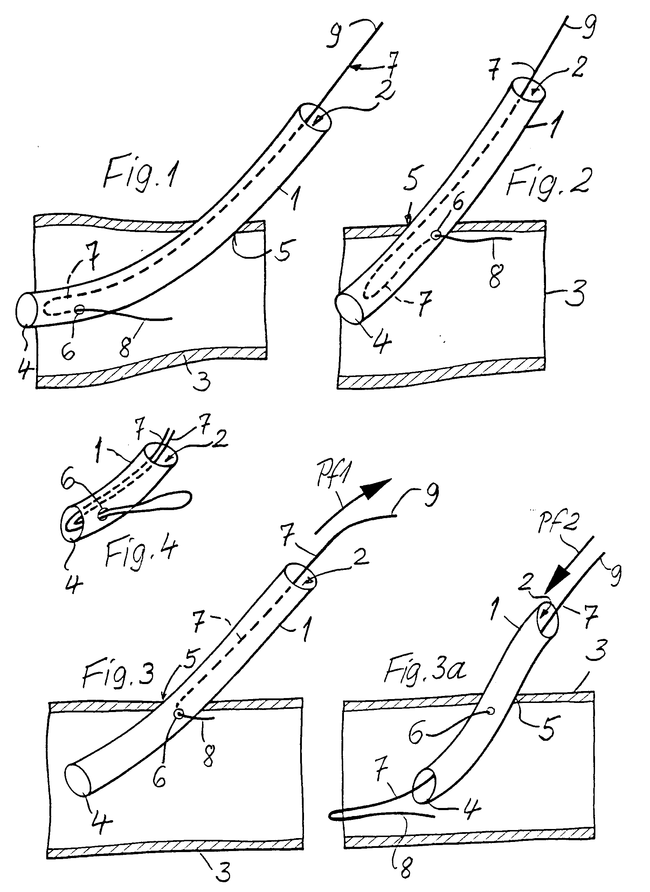

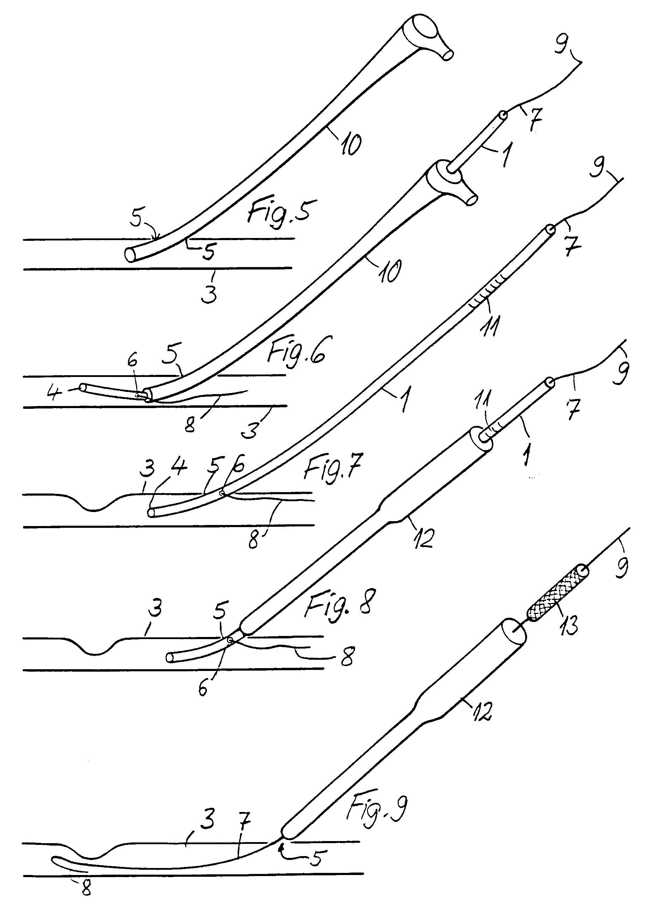

In den Figuren 1 bis 4 ist eine Leitung 1 mit einer Innenlängshöhlung 2, zum Beispiel ein Schlauch oder ein Katheter bezüglich ihrer Verankerung in einem Blutgefäß 3 dargestellt. Spezielle Anwendungsbeispiele und Ausgetaltungen dieser Anordnung sind in zwei Ausführungsbeispielen, nämlich einem Ausführungsbeispiel gem. Fig.5 bis 9 und einem weiteren Ausführungsbeispiel gem. Fig.10 bis 14 im folgenden näher erläutert. Dabei erhalten übereinstimmende Teile auch bei unterschiedlicher Gestaltung übereinstimmende Bezugszahlen.

Anhand der Figuren 2,7 und 8 sowie 11 und 12 erkennt man, daß zwischen der Mündung 4 der Leitung 1 und ihrem Eintritt 5 in das Blutgefäß 3 ein vorbestimmter Abstand vorgesehen ist. Dieser wird dadurch erreicht, daß die Leitung 1 in diesem Abstand von ihrer Mündung 4 eine seitliche Lochung 6 oder Öffnung hat und daß zum Fixieren der Leitung 1 in der gewünschten Position ein im Bereich der Mündung 4 U-förmig gekrümmter Faden 7 aus federelastischem Werkstoff, also mit einer gewissen Eigensteifigkeit vorgesehen ist, der mit einem Halteende 8 durch die seitliche Lochung 6 austritt. Dieser Faden 7 wird von dem zwischen der Lochung 6 und der Mündung 4 liegenden Bereich der Leitung 1 in seiner U- oder V-förmigen Position zusammengehalten, so daß das austretende Halteende im Bereich der Lochung zunächst entgegen der Richtung umgelenkt wird, in welcher eine Zugbelastung auf die Leitung 1 auftreten könnte. Dadurch wird sichergestellt, daß das Halteende 8 des Fadens 7 auch bei einem Zug auf die Leitung 1 seine Position behält und als Widerhaken oder borstenförmige Verankerung an der Innenseite des Blutgefäßes 3 wirken kann. Das andere Ende 9 des Fadens steht an dem außerhalb des Blutgefäßes befindlichen Bereich der Leitung 1 über, so daß zum Lösen der Verankerung gem. der Fig.3 an diesem Ende in Richtung des Pfeiles Pf.1 gezogen werden kann, wodurch sich die U- oder V-förmige Schlinge im Mündungsbereich auflöst und dann auf das Halteende 8 zurückgezogen werden kann. Gemäß Fig.3a kann der Faden 7 zum Lösen aus seiner Halteposition auch zunächst gemäß dem Pfeil Pf2 nach innen verschoben werden, bis das Halteende 8 die Lochung 6 und zweckmäßigerweise auch die Mündung 4 der Leitung 1 verlassen hat; danach kann die Leitung 1 zurückgezogen und der Faden 7 noch als Führung verwendet oder der Faden 7 zurückgezogen werden.

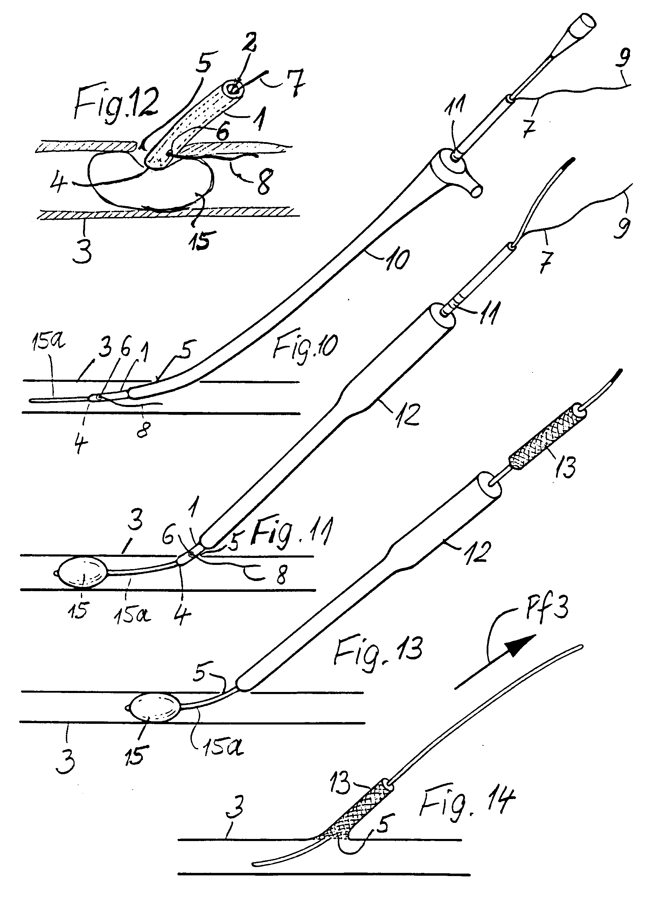

In Fig.4 erkennt man, daß der Faden 7 über seine Länge und außerhalb der Leitung 1 und der seitlichen Lochung 6 doppelt verlaufen kann, wobei er in diesem Ausführungsbeispiel an seinem Halteende 8 U- oder V-förmig umgebogen oder geknickt ist, während im Mündungsbereich dann beide Faden-Lagen die schon erwähnte U- oder V-förmige Verformung haben, damit auch in diesem Falle das Halteende 8 von der seitlichen Lochung 6 aus zunächst in Richtung zu der Mündung 4 verläuft. Dies ergibt eine noch stabile Halterung der Leitung 1 bzw. eines entsprechenden Katheters.

Dieses Verankerungsprinzip gem. den Figuren 1 bis 4 ist beim Ausführungsbeispiel nach Fig.5 bis 9 und auch nach dem Ausführungsbeispiel nach Fig.10 bis 14 jeweils angewendet. Zum Einführen und Verankern der Leitung 1 dient dabei gem. Fig.5 ein an sich bekanntes Führungsbesteck 10, durch welches gem. Fig.6 die in diesem Falle als Katheter ausgebildete Leitung 1 mit der Fadenschlinge eingeführt wird. Nach Entfernen des Führungsbesteckes 10 bleibt die Leitung 1 bzw. der Katheter gemäß Fig.7 in dem Blutgefäß 3 hängen, weil das Halteende 8 des Fadens 7 sich an die Innenseite des Blutgefäßes 3 anlegt. Dabei erkennt man, daß der Katheter bzw. die Leitung 1 eine Längenmarkierung 11 trägt. Falls eine solche Markierung 11 nicht von vorneherein vorhanden ist, könnte sie auch vom Operateur in dem Bereich angebracht werden, der in Gebrauchsstellung etwa am Durchtritt der Leitung 1 durch die Hautoberfläche liegt.

Diese Längenmarkierung 11 erlaubt gem. Fig.8 das Aufschieben einer Einführhülse 12, bis deren Mündung kurz vor dem Eintritt 5 in das Blutgefäß 3 steht. Nunmehr kann gem. Fig.3 oder 3a der Faden 7 zurückgezogen und die Leitung 1 bzw. der Katheter entfernt werden. Danach kann ein Verschlußstöpsel 13 über den noch in das Blutgefäß 3 ragenden und etwas darin eingeschobenen Faden 7 oder einen zusätzlichen durch die Leitung 7 einführbaren Draht bis vor den Eintritt 5 in das Blutgefäß 3 geführt werden. Somit kann die als Eintritt 5 dienende Punktionsöffnung beispielsweise eine Arterie sicher verschlossen werden, wie es auch in Fig.14 dargestellt ist.

Beim Ausführungsbeispiel gem. Fig.10 bis 14 ist eine Leitung 1 bzw. ein Katheter mit einer Abdichtung des Eintrittsbereiches gegenüber dem Eintritt 5 in das Blutgefäß 3 dargestellt. Gemäß Fig.10 wird dabei die Leitung 1 wiederum mit einem Führungsbesteck 10 in das Blutgefäß 3 eingeführt. Im Inneren der Leitung 1 ist ein lösbar oder entnehmbar angeordneter Ballonkatheter 15a angeordnet, dessen Ballon 15 in Gebrauchsstellung wenigstens teilweise aus der in dem Hohlorgan bzw. dem Blutgefäß 3 befindlichen Mündung 4 austritt.

Nach der schon beim vorhergehenden Ausführungsbeispiel beschriebenen, in Fig.11 dargestellten Verankerung der Leitung 1 mit Hilfe des Fadens 7 kann der Ballon 15 gem. Fig.11 stromaufwärts entfaltet werden und so die Blutzufuhr zu dem Eintritt 5 unterbinden, bis dort ein Kollagenverschluß 13 angeordnet ist. Dabei entspricht das Einführen des Kollagenstöpsels 13 gem. Fig.13 der schon in Fig.9 dargestellten Arbeitsweise. Auch eine Längenmarkierung 11 kann gem. Fig. 11 vorhanden sein.

In Fig.12 ist dargestellt, daß der Ballon 15 auch unmittelbar unterhalb des Eintrittes 5 und an der Mündung 4 der Leitung 1 aufgefaltet werden kann, um an dieser Stelle eine vorübergehende Abdichtung zu erzeugen, bis der Kollagenverschluß 13 nach einem Aufschieben über den Ballonkatheter gem. Fig. 14 seine Gebrauchsstellung erreicht hat. Nach Entleeren des Ballons 15 kann dieser dann gem. dem Pfeil Pf 3 in Fig.14 durch den röhrchenförmig ausgebildeten Kollagenverschluß 13 hindurch zurückgezogen werden, wonach das Kollagen in bekannter Weise selbsttätig zu einem abdichtenden Verschluß wird.

Die Leitung 1, die ein Schlauch und insbesondere ein Katheter sein kann, kann dadurch in einem Hohlorgan und insbesondere in einem Blutgefäß 3 mit einem vorbestimmten Abstand ihrer Mündung 4 von ihrem Eintritt 5 in das Hohlorgan verankert werden, daß sie etwa in dem Abstand ihrer Verankerungstiefe eine seitliche Lochung 6 und in ihrer Innenlängshöhlung 2 einen Verankerungs-Faden 7 aus federelastischem Werkstoff hat. Der Faden 7 ist im Bereich der Mündung 4, also in Einführrichtung hinter der Lochung 6 bzw. in dem Bereich zwischen dieser Lochung 6 und der Mündung 4 U-förmig gekrümmt und tritt mit einem Halteende 8 durch die seitliche Lochung 6 aus. Der zwischen der Lochung 6 und der Mündung 4 liegende Bereich der Leitung 1 wird in seiner U- oder V-förmigen Lage zusammengehalten, wobei die eigentliche Krümmung unter Umständen sogar vor oder außerhalb der Mündung 4 liegen könnte. Das dem Halteende 8 entgegengesetzte andere Ende 9 des Fadens 7 ragt aus dem außerhalb des Hohlorganes befindlichen Bereich der Leitung 1 hervor, so daß zum Lösen der Verankerung an diesem Ende 9 so lange gezogen werden kann, bis die U- oder V-förmige Schlinge aufgelöst und das Halteende 8 zurückgezogen sind. Gegebenenfalls kann dabei der Faden auch doppelt liegen und an seinem Halteende 8 umgebogen sein, so daß sich im Mündungsbereich zwei schlingenförmige Lagen ergeben.

## Patentansprüche

1. Leitung (1) mit einer Innenlängshöhlung (2), insbesondere Schlauch, zum Einführen in ein Hohlorgan, z.B. in ein Blutgefäß (3), in die Harnblase oder dergleichen, wobei zwischen der Mündung (4) der Leitung (1) und ihrem Eintritt (5) in das Hohlorgan in Gebrauchsstellung ein vorbestimmter Abstand vorgesehen ist, **dadurch gekennzeichnet**, daß die Leitung (1) etwa in dem vorbestimmten Abstand von ihrer Mündung (4) eine Öffnung (6) hat, und daß zum axialen Fixieren der Leitung (1) im Eintritt (5) ein elastisches Element (7) vorgesehen ist, das durch die Öffnung (6) hindurch sich im Hohlorgan (3) abstützt und das durch eine axiale Relativbewegung zwischen Leitung (1) und elastischem Element (7) die Leitung (1) gegenüber dem Hohlorgan (3) wieder freigibt.

2. Leitung nach Anspruch 1, dadurch gekennzeichnet, daß die Leitung (1) etwa in dem Abstand von ihrer Mündung (4), mit dem sie in Gebrauchsstellung in das Hohlorgan ragt, wenigstens eine seitliche Lochung (6) oder Öffnung hat und daß zum Fixieren der Leitung (1) ein im Bereich der Mündung (4) der Leitung U-förmig gekrümmter Faden (7) aus federelastischem Werkstoff vorgesehen ist, der mit einem Halteende (8) durch die seitliche Lochung (6) austritt, von dem zwischen dieser Lochung (6) und der Mündung (4) liegenden Bereich der Leitung (1) U-oder V-förmig liegend zusammengehalten ist und mit dem anderen Ende (9) an dem außerhalb des Hohlorganes befindlichen Bereich der Leitung (1) übersteht.

3. Leitung nach Anspruch 1 oder 2, dadurch gekennzeichnet, daß der Faden (7) über seine Länge und außerhalb der Leitung (1) und der seitlichen Lochung (6) doppelt verläuft und an seinem Halteende (8) insbesondere U- oder V-förmig umgebogen oder geknickt ist.

4. Leitung nach Anspruch 1 bis 3, dadurch gekennzeichnet, daß mehrere seitliche Lochungen in unterschiedlichen Abständen zur Mündung vorgesehen sind.

5. Leitung nach einem der Ansprüche 1 bis 4, dadurch gekennzeichnet, daß mehrere seitliche Lochungen etwa in gleichem Abstand am Umfang verteilt und/oder in unterschiedlichen Abständen zur Mündung vorgesehen sind.

6. Leitung nach einem der Ansprüche 1 bis 5, dadurch gekennzeichnet, daß ihre Außenseite markierbar ist oder eine Längenmarkierung (11) trägt.

7. Leitung nach einem der Ansprüche 1 bis 6, dadurch gekennzeichnet, daß sie mehrere Verankerungsfäden enthält, von denen jeweils ein Halteende durch dieselbe oder unterschiedliche seitliche Lochungen austreten.

8. Leitung mit einer Abdichtung ihres Eintrittsbereiches gegenüber dem Eintritt (5) in das Hohlorgan, nach einem der Ansprüche 1 bis 7, dadurch gekennzeichnet, daß sie in ihrem Inneren einen lösbar oder entnehmbar angeordneten Ballonkatheter (15a) enthält, dessen Ballon (15) in Gebrauchsstellung wenigstens teilweise aus der in dem Hohlorgan befindlichen Mündung (4) austritt.
